# EUROPÄISCHE PATENTANMELDUNG

(11) **EP 3 075 721 A1**
(43) Veröffentlichungstag der Anmeldung: **05.10.2016**
(21) Anmeldenummer: 15161579.6
(22) Anmeldetag: 30.03.2015
(51) Int. Cl.: C07C 209/48, B01J 25/00, B01J 35/08, B01J 35/02

(54) **Verfahren zur Herstellung von 3-Aminomethyl-3,5,5-trimethylcyclohexylamin**

(71) Anmelder: Evonik Degussa GmbH, 45128 Essen (DE)
(72) Erfinder: Kohlstruck, Dr. Stephan, 45966 Gladbeck (DE); Rittsteiger, Anne, 59399 Olfen (DE); Rüfer, Dr. Alexander Martin, 45657 Recklinghausen (DE); Schlueter, Norbert, 48712 Gescher (DE); Schneider, Sven, 45711 Datteln (DE); Sowka, Sabrina, 48249 Dülmen (DE); Streukens, Dr. Guido, 42111 Wuppertal (DE); Röder, Stefan, 36391 Sinntal (DE); Berweiler, Monika, 63477 Maintal (DE)

(57) **Zusammenfassung**

Verfahren zur Herstellung von Isophorondiamin, dadurch gekennzeichnet, dass
A) Isophoronnitril direkt in einer Stufe in Anwesenheit von Ammoniak, Wasserstoff, eines Hydrierkatalysator und gegebenenfalls weiteren Zusätzen und in An- oder Abwesenheit von organischen Lösungsmitteln aminierend zu Isophorondiamin hydriert wird;
oder
B) Isophoronnitril in mindestens zwei oder mehreren Stufen zunächst ganz oder teilweise in Isophoronnitrilimin umgewandelt wird, und dieses Isophoronnitrilimin als Reinsubstanz, oder im Gemisch mit anderen Komponenten und/oder Isophoronnitril, unter Anwesenheit von mindestens Ammoniak, Wasserstoff und eines Katalysators aminierend zu Isophorondiamin hydriert wird;

wobei der Katalysator nach der Aktivierung in seiner Gesamtheit die folgende Zusammensetzung aufweist, in Gewichtsprozent (Gew.-%), wobei sich die Anteile zu 100 Gew.-% addieren, bezogen auf die enthaltenden Metalle:
Cobalt: 55 bis 95 Gew.-%
Aluminium: 5 bis 45 Gew.-%
Chrom: 0 bis 3 Gew.-%
Nickel: 0 bis 7 Gew.-%;

und der Katalysator in Form von Hohlkugeln vorliegt, mit Durchmessern von 1 bis 8 mm.

## Beschreibung

Die Erfindung betrifft ein verbessertes Verfahren zur Herstellung von 3-Aminomethyl-3,5,5-trimethylcyclohexylamin, nachfolgend Isophorondiamin oder abgekürzt IPDA genannt, mittels katalytischer Hydrierung und/oder katalytischer reduktiver Aminierung (auch als aminierende Hydrierung bezeichnet) von 3-Cyano-3,5,5-trimethylcyclohexanon, nachfolgend Isophoronnitril oder abgekürzt IPN genannt.

Die Herstellung von IPDA durch aminierende Hydrierung von IPN ist bekannt und bereits mehrfach beschrieben worden.

Im einfachsten Fall (US 3,352,913) wird IPN in Gegenwart von Wasserstoff und eines Überschusses an Ammoniak an einem Cobaltkatalysator zur Reaktion gebracht. Zunächst bildet sich aus IPN und Ammoniak durch Wasserabspaltung das Isophoronnitrilimin, IPNI, das nachfolgend zum IPDA hydriert wird.

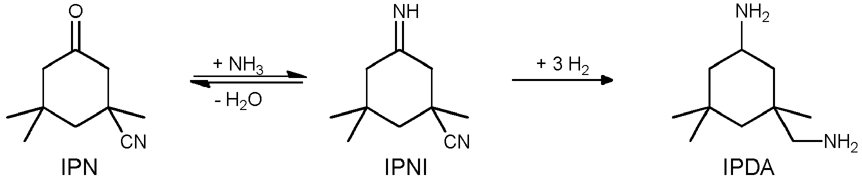

Die Ausbeute an IPDA wird bei dieser Art der Reaktionsführung maßgeblich durch den Überschuss an Ammoniak bestimmt. Die maximal erzielten IPDA-Ausbeuten liegen bei ca. 80 %. Hauptnebenprodukt ist der sogenannte Aminoalkohol, IPAA, der aus der direkten Hydrierung des IPN resultiert.

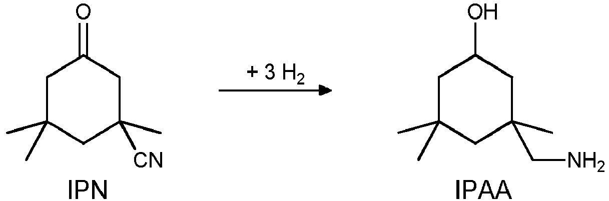

Eine wesentliche Steigerung der IPDA-Ausbeute wird erreicht, wenn die Bildung des IPNI durch Einsatz geeigneter Iminierungskatalysatoren beschleunigt wird. Als Iminierungskatalysatoren sind z. B. saure Ionenaustauscherharze (EP 042 119) geeignet. Daneben können auch acide Metalloxide (EP 449 089), sulfonsäuregruppenhaltige Organopolysiloxane (EP 816 323), Heteropolysäuren (DE 44 26 472) und Aktivkohle (EP 061 137) als Iminierungskatalysatoren eingesetzt werden. Neben der Reduzierung des unerwünschten Aminoalkohols werden auch andere Nebenprodukte deutlich zurück gedrängt, z. B. bicyclische Verbindungen und solche Nebenprodukte, die aus der Abspaltung von HCN resultieren.

Auf die Problematik der Abspaltung von HCN aus gamma-Ketonitrilen, wie dem IPN, wird in der Literatur besonders hingewiesen (US 3,352,913). Zum einen wird bemerkt, dass durch die HCN-Abspaltung die Ausbeute an IPDA reduziert wird (EP 042 119, DE 44 26 472).

Zum anderen wird darauf hingewiesen, dass HCN als Katalysatorgift wirkt und zu einer Desaktivierung des Hydrierkatalysators führt (EP 394 967 A1, Seite 2 Zeile 34 ff, Seite 3 Zeile 44 ff). Es wird daher empfohlen, den Iminierungsschritt so durchzuführen, dass möglichst kein HCN abgespalten wird.

Gemäß EP 913 387 können zur Selektivitätssteigerung bei der Herstellung von IPDA auch quaternäre Ammoniumbasen eingesetzt werden. Entsprechend modifizierte Katalysatoren weisen speziell bei Verwendung eines Lösungsmittels eine deutlich höhere Standzeit auf als alkalimodifizierte Katalysatoren.

Weiterhin sind Verfahren zur Herstellung von Isophorondiamin aus CN 104230721A, EP 2649042A und WO 2012126869A bekannt.

In dem Dokument DE 199 33 450.1 werden Metallkatalysatoren beschrieben, die in Form von Hohlkugeln eine niedrige Schüttdichte von 0,3 bis 1,3 g/ml besitzen. Neben den Katalysatoren wird außerdem deren Anwendung in Hydrierreaktionen beansprucht.

Ein Verfahren zur Herstellung von Isophorondiamin ist aus der WO 2002051791 bekannt. Es werden Katalysatoren in Form von Hohlkugeln verwendet.

Die zugrundeliegende Aufgabe war es, ein Verfahren zur Herstellung von 3-Aminomethyl-3,5,5-trimethylcyclohexylamin durch Hydrierung von Isophoronnitril zu finden, wobei die Ausbeute und Selektivität bei der reduktiven Aminierung von Isophoronnitril zur Herstellung von Isophorondiamin verbessert werden sollte.

Überraschend wurde ein neuer Katalysator wie unten näher beschrieben, gefunden. Als zusätzlicher unerwarteter Effekt konnten eine höhere Aktivität, wodurch niedrigere Reaktionstemperaturen möglich sind, und eine bessere Langzeitstabilität des Katalysators festgestellt werden.

Überraschend wurde nun gefunden, dass der erfindungsgemäße Katalysator, bestehend aus Hohlkugeln mit bestimmten Durchmessern, hergestellt aus einer Metall-Legierung nach Aktivierung durch Laugen, die Aufgabe der Erfindung löst und zusätzlich eine höhere Aktivität und eine bessere Langzeitstabilität des Katalysators festgestellt wurden.

Gegenstand der Erfindung ist ein Verfahren zur Herstellung von Isophorondiamin dadurch gekennzeichnet, dass
A) Isophoronnitril direkt in einer Stufe in Anwesenheit von Ammoniak, Wasserstoff, eines Hydrierkatalysators und gegebenenfalls weiteren Zusätzen und in An- oder Abwesenheit von organischen Lösungsmitteln aminierend zu Isophorondiamin hydriert wird;
   oder
B) Isophoronnitril in mindestens zwei oder mehreren Stufen zu Isophorondiamin umgesetzt wird, wobei zunächst Isophoronnitril ganz oder teilweise in Isophoronnitrilimin umgewandelt wird, und dieses Isophoronnitrilimin als Reinsubstanz, oder im Gemisch mit anderen Komponenten und/oder Isophoronnitril, unter Anwesenheit von mindestens Ammoniak, Wasserstoff und eines Katalysators aminierend zu Isophorondiamin hydriert wird;
   wobei der Katalysator die folgenden Eigenschaften aufweist:
   I. Der Katalysator weist nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:
      - Cobalt:: 55 bis 95 Gew.-%
      - Aluminium:: 5 bis 45 Gew.-%
      - Chrom:: 0 bis 3 Gew.-%
      - Nickel:: 0 bis 7 Gew.-%
      und
   II. Der Katalysator liegt in Form von Hohlkugeln vor mit Durchmessern von 1 bis 8 mm.

Das Katalysatormaterial besteht aus einer Metall-Legierung, wobei die Metall-Legierung durch Basen auf der Oberfläche aktiviert ist. Die Schichtdicke der aktivierten Schicht auf der Partikeloberfläche des Katalysators beträgt bevorzugt 50 bis 1500 Mikrometer (µm). Sie kann aber auch größer oder kleiner sein. Auf der Oberfläche befindet sich demnach die katalytisch aktive Zusammensetzung des Katalysators. Es ist aber auch im Rahmen der Erfindung möglich, das gesamte Katalysator-Partikel fast vollständig oder vollständig auszulaugen.

Der erfindungsgemäße Katalysator liegt nach der Aktivierung als Hohlkugeln vor.

Das erfindungsgemäße Katalysatormaterial weist nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:
1. Variante
   - Cobalt:: 55 bis 95 Gew.-%
   - Aluminium:: 5 bis 45 Gew.-%
   - Chrom:: 0 bis 3 Gew.-%
   - Nickel:: 0 bis 7 Gew.-%
   und/oder
2. Variante
   - Cobalt:: 55 bis 90 Gew.-%
   - Aluminium:: 5 bis 44,5 Gew.-%
   - Chrom:: 0,5 bis 5 Gew.-%
   und/oder
3. Variante
   - Cobalt:: 55 bis 88 Gew.-%
   - Aluminium:: 5 bis 44,5 Gew.-%
   - Nickel:: 0,5 bis 7 Gew.-%
   und/oder
4. Variante
   - Cobalt:: 55 bis 85 Gew.-%
   - Aluminium:: 5 bis 43,5 Gew.-%
   - Chrom:: 0,5 bis 3 Gew.-%
   - Nickel:: 1 bis 7 Gew.-%
   und/oder
5.Variante
   - Cobalt:: 57 bis 84 Gew.-%
   - Aluminium:: 10 bis 40 Gew.-%
   - Chrom:: 1 bis 2 Gew.-%
   - Nickel:: 2 bis 4 Gew.-%

Gesamtheit bedeutet, dass bei der Zusammensetzung nicht zwischen dem Gehalt der Metalle auf der Oberfläche und in der aktivierten Schicht und im Kern der Katalysator-Partikel unterschieden wird, sondern Alles zusammen addiert und berechnet wird.

Der Katalysator liegt in Form von Hohlkugeln vor.

Zusätzlich weist der erfindungsgemäße Hohlkugel Katalysator nach der Aktivierung die folgenden Durchmesser auf:
Im Allgemeinen kann der Durchmesser des Katalysators, also die Hohlkugeln, Durchmesser von 1 bis 8 Millimeter (mm) aufweisen.
In einer ersten bevorzugten Variante der Erfindung variieren die Durchmesser des Katalysators, also die Hohlkugeln von 2,5 bis 5,5 Millimeter (mm).
In einer zweiten bevorzugten Variante der Erfindung variieren die Durchmesser des Katalysators, also die Hohlkugeln von 3 bis 8 Millimeter (mm).
In einer dritten bevorzugten Variante der Erfindung variieren die Durchmesser des Katalysators, also die Hohlkugeln von 1 bis 3 Millimeter (mm).

Das für die Herstellung des erfindungsgemäßen Katalysators verwendete Legierungspulver hat eine bevorzugte Korngröße von 5 bis 150 Mikrometer (µm). Die Korngröße kann aber auch kleiner oder größer gewählt werden.

Die Bestimmung der Partikelgrößen wird in der DIN ISO 9276-1 (September 2004) und 9276-2 (Februar 2006) und 9276-4 (Februar 2006) und 9276-6 (Januar 2012) beschrieben. Außerdem findet man genaue Angaben zur Definition von Partikelgrößen, der Verteilung von Partikelgrößen und der Messung von Partikelgrößen in HORIBA® Scientific, A GUIDEBOOK TO PARTICLE SIZE ANALYSIS, 2012, von HORIBA® Instruments, Inc, Irvine, USA.

Erfindungsgemäß kann die Verteilung der Partikelgrößen und die Messung der Partikelgrößen durch Laserverfahren (ISO 13320, 2012), Lichtverfahren oder Bildverfahren bestimmt werden. Geeignete Methoden und Beschreibungen zur Siebanalyse sind beschrieben in: DIN 66165-1:1987-04 Partikelgrößenanalyse; Siebanalyse; Grundlagen, und in DIN 66165-2:1987-04 Partikelgrößenanalyse; Siebanalyse; Durchführung.

Paul Schmidt, Rolf Körber, Matthias Coppers: Sieben und Siebmaschinen: Grundlagen und Anwendung. Wiley-VCH Verlag, 2003, ISBN 9783527302079, Kapitel 4.4: Analysesiebung. Jörg Hoffmann: Handbuch der Messtechnik. Hanser Verlag, 2007, ISBN 978-3-446-40750-3, Kapitel 3.12.16.2.1.

Der erfindungsgemäße Hohlkugel Katalysator weist besonders bevorzugt nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle (vierte bevorzugte Variante):
- Cobalt:: 57 bis 84 Gew.-%
- Aluminium:: 10 bis 40 Gew.-%
- Chrom:: 1 bis 2 Gew.-%
- Nickel:: 2 bis 4 Gew.-%
und mit
Durchmesser des Hohlkugel Katalysators, also der Hohlkugeln mit einer statistischen Verteilung zwischen 2,5 bis 5,5 Millimeter (mm),
und/oder
Durchmesser des Hohlkugel Katalysators, also der Hohlkugeln mit einer statistischen Verteilung zwischen 3,5 bis 6,5 Millimeter (mm),
und/oder
Durchmesser des Hohlkugel Katalysators, also der Hohlkugeln mit einer statistischen Verteilung zwischen 1 bis 3 Millimeter (mm),
und/oder
Durchmesser des Hohlkugel Katalysators, also der Hohlkugeln mit einer statistischen Verteilung zwischen 3 bis 8 Millimeter (mm),
wobei bis zu 10 Prozent der Partikel auch außerhalb des genannten Bereichs der genannten Untergrenze oder Obergrenze, aber auch jeweils bis zu 10 Prozent außerhalb des genannten Bereichs der genannten Untergrenze und Obergrenze, liegen können.

Der erfindungsgemäße Katalysator kann aus einer oder mehreren Schichten aufgebaut sein. Die Schichten können dabei sowohl aus dem gleichen, als auch aus unterschiedlichen Katalysatormaterialen, also Legierungspulvern, bestehen. Legierungspulver können sich dabei in der Zusammensetzung der Metalle und/oder der Korngröße unterscheiden. Durch die Verwendung von feinem Legierungspulver wird ein kompakter Schalenaufbau mit geringer Porosität und einer hohen mechanischen Stabilität erhalten. Grobes Legierungspulver dagegen ergibt einen porösen Aufbau der Hohlkugelschale. Diese führt zu einer Vergrößerung der aktiven Oberfläche der erfindungsgemäßen Hohlkugeln.

Über die Zusammensetzung des Legierungspulvers kann die Aktivität und/oder Selektivität des erfindungsgemäßen Katalysators beeinflusst werden.

Außerdem ist es möglich, dass der Hohlkugelförmige Katalysator eine bimodale oder multimodale Verteilung in Bezug auf die Durchmesser der Hohlkugeln aufweist. Bei einer bimodalen Verteilung werden demnach Hohlkugeln mit zwei verschiedenen Durchmessern eingesetzt, bei einer multimodalen Verteilung Hohlkugeln mit mindestens drei verschiedenen Durchmessern. Bevorzugt werden die Hohlkugeln gemäß der ersten bis dritten Variante und/oder Hohlkugeln gemäß der vierten Variante ausgewählt.

Außerdem ist es auch möglich, dass der Katalysator aus einer oder mehreren Schichten aufgebaut ist und/oder die Schichten dabei sowohl aus dem gleichen und/oder unterschiedlichen Katalysatormaterialen, also Legierungspulvern, bestehen, und/oder, dass der Hohlkugelförmige Katalysator eine bimodale oder multimodale Verteilung in Bezug auf die Durchmesser der Hohlkugeln aufweist.

Der dieser Erfindung zugrunde liegende Vorteil wird durch die Verwendung von Katalysatoren in Form von Hohlkugeln erreicht. Die Herstellung der in dem erfindungsgemäßen Verfahren angewendeten Katalysatoren kann entsprechend der in DE 199 33 450.1 beschriebenen Methode durchgeführt werden. Nach dieser Methode wird eine Mischung eines Legierungspulvers aus katalytisch aktiven Metallen mit dem auslaugbaren Metall Aluminium, einem organischen Binder und gegebenenfalls einem anorganischen Binder, Wasser und Promotoren auf Kugeln, die aus einem thermisch zersetzbaren Material bestehen, aufgetragen. Bevorzugt können Polystyrolschaumkugeln verwendet werden. Das Auftragen der die Metall-Legierung enthaltenden Mischung auf die Polymerkugeln kann bevorzugt in einem Wirbelbett durchgeführt werden. Als organische Binder können bevorzugt 0 bis 10 Gew.-% Polyvinylalkohol und/oder 0 bis 3 Gew.-% Glycerin eingesetzt werden. Die beschichteten Polymerschaumkugeln werden anschließend oberhalb 300 °C, bevorzugt in einem Bereich zwischen 450 und 1300 °C calziniert, um den Polymerschaum thermisch zu entfernen und das Metall zu sintern. Dadurch erhalten die Hohlkugeln eine stabile Form. Nach der Calzinierung werden die Hohlkugelförmigen Katalysatoren durch Behandeln mit basischen Lösungen, bevorzugt Alkali- oder Erdalkalihydroxide in Wasser, noch bevorzugter wässrige Natronlauge aktiviert. Die so erhaltenen Katalysatoren besitzen Schüttdichten zwischen 0,3 und 1.3 kg/l.

Erfindungsgemäß besitzen die in dem Verfahren angewendeten Katalysatoren die Form von Hohlkugeln. Hohlkugeln sind üblicherweise leicht herzustellen und besitzen eine hohe Bruchfestigkeit.

Die erfindungsgemäß angewendeten, Hohlkugelförmigen Katalysatoren können einen Binder enthalten. Der Binder ermöglicht eine größere Festigkeit der Katalysator Hohlkugel, die bedingt durch ihre hohle Form nötig ist. Bevorzugt werden Pulver der Metalle, die auch als katalytisch aktive Bestandteile in der Katalysatorlegierung enthalten sind, bei der Herstellung der Katalysator Hohlkugel als Binder zugesetzt. Es ist aber auch möglich, andere Binder, insbesondere andere Metalle als Binder zuzusetzen. Bevorzugt wird kein Binder eingesetzt. Hohlkugelförmige Kobaltkatalysatoren besitzen auch ohne zugesetzten Binder eine ausreichende Festigkeit.

In dem erfindungsgemäßen Verfahren können mit anderen Metallen dotierte Hohlkugelförmige Raneykatalysatoren verwendet werden. Die Dotierungsmetalle werden oftmals auch als Promotoren bezeichnet. Das Dotieren von Raney- Katalysatoren wird beispielsweise in den Dokumenten US 4,153,578, DE 21 01 856, DE 21 00 373 oder DE 20 53 799 beschrieben. Bevorzugte Elemente zum Dotieren sind Elemente der Gruppen 1A, 2A, 3B bis 7B, 8, 1B, 2B und 3A des Periodensystems sowie Germanium, Zinn, Blei, Antimon und Wismut. Besonders bevorzugt sind Mangan, Eisen, Vanadium, Tantal, Titan, Wolfram, Molybdän, Rhenium und/oder Metalle der Platingruppe. Der Anteil an Promotoren im Katalysator kann bevorzugt 0 bis 5 Gew.-% betragen. Die Promotoren können bereits als Legierungsbestandteil enthalten sein, oder erst zu einem späteren Zeitpunkt, insbesondere nach der Aktivierung zugegeben werden.

Während der Herstellung des erfindungsgemäßen Katalysators ist eine oder mehrere Calzinierungen bei Temperaturen oberhalb von 300 °C, bevorzugt in einem Bereich zwischen 450 und 1300 °C erforderlich. Wird dieser Vorgang unter einer Sauerstoffhaltigen Atmosphäre durchgeführt, kann Sauerstoff in die Katalysatorschicht eingebunden werden. Der Anteil im Katalysator ist dabei von der Dauer der Calzinierung und/oder den Aktivierungsbedingungen abhängig und kann bevorzugt 0 bis 25 Gew.-% betragen.

Für den Fall, dass derartige Elemente enthalten sind, in einer Menge von maximal ca. 30 Gew.-%, reduziert sich der Anteil der oben genannten Metall Co und Al sowie gegebenenfalls Cr und Ni im Katalysator entsprechend, wobei die Anteile an Co und Al sowie gegebenenfalls Cr und Ni dann nach der Aktivierung sich zu mindestens 70 Gew.-% addieren, bezogen auf die enthaltenden Metalle.

In dem erfindungsgemäßen Verfahren werden Hohlkugelförmige Katalysatoren mit einem Durchmesser von 1 bis 8 mm und eine Schalendicke von 50 bis 1500 Mikrometer (µm) verwendet. Die Katalysatorschalen können undurchlässig sein, oder eine Porosität von 80% und höher aufweisen.

In dem erfindungsgemäßen Verfahren können Hohlkugelförmige Katalysatoren verwendet werden, die aus einer oder aus mehreren Schichten bestehen. Haben die Katalysatorkörper mehrere Schichten, werden die Katalysatorkörper bei der Herstellung zwischen den einzelnen Beschichtungsschritten getrocknet. Dieses wird bevorzugt im Wirbelbett bei Temperaturen von 60 bis 150 °C durchgeführt. Es ist auch möglich einen Hohlkugelförmigen Katalysator mit mehreren Schichten herzustellen, wobei nicht zwischen den einzelnen Beschichtungsschritten getrocknet wird.

Während des erfindungsgemäßen Verfahrens werden die Hohlkugelförmigen Katalysatoren in der aktivierten Form eingesetzt. Das in den nicht aktivierten Katalysatorkörpern vorhanden auslaugbare Metall kann im aktivierten Zustand ganz oder nur teilweise mit Alkalien herausgelaugt worden sein.

Allgemeine Methode zur Herstellung des Katalysators:
a) Herstellung der Legierung
   Die Herstellung der Legierung erfolgt thermisch, zum Beispiel in einem Induktionsofen. Es werden dabei die Metalle geschmolzen und eine Legierung erhalten. Die fertige Schmelze wird für die Weiterverarbeitung zum Beispiel zu Barren gegossen.
b) Herstellung der Pulver
   Die Legierung wird in geeigneten Geräten zu Pulver verarbeitet, zum Beispiel über einen Backenbrecher vorzerkleinert und über eine Kugel- oder Stabmühle weiter gemahlen. Durch einen optionalen Siebschritt kann die gewünschte Größenverteilung der Partikel durch die Wahl der entsprechenden Siebe erhalten werden.
c) Herstellung der Hohlkugeln
   Für die Herstellung der Hohlkugeln wird eine Mischung aus Legierungspulver, einem organischen und gegebenenfalls anorganischen Binder, Wasser und Promotoren auf Kugeln, die aus einem thermisch zersetzbaren Material bestehen, aufgetragen. Bevorzugt können Polystyrolschaumkugeln verwendet werden. Das Auftragen der die Metall-Legierung enthaltenden Mischung auf die Polymerkugeln kann bevorzugt in einem Wirbelbett durchgeführt werden. Als organische Binder können bevorzugt 0 bis 10 Gew.-% Polyvinylalkohol und/oder 0 bis 3 Gew.-% Glycerin eingesetzt werden. Die beschichteten Polymerschaumkugeln werden anschließend oberhalb von 300 °C, bevorzugt in einem Bereich zwischen 450 und 1300 °C calziniert, um den Polymerschaum thermisch zu entfernen und das Metall zu sintern. Dadurch erhalten die Hohlkugeln eine stabile Form.
   d) Aktivierung des Katalysators
   Die Aktivierung des Katalysators erfolgt in geeigneten Apparaturen. Es können dabei organische oder anorganische Basen eingesetzt werden. Bevorzugt wird eine Lauge (z. B. Natronlauge), verwendet, wobei durch einen exothermen Vorgang ein Teil des Aluminiums unter Bildung von Wasserstoff und Aluminatlauge aus der Legierung herausgelöst wird. Die Konzentration der Lauge kann zwischen 5 und 30 Gew.-% liegen und die Reaktionstemperatur zwischen 50 und 110 °C. Der Grad der Aktivierung wird über die Temperatur und die Reaktionszeit bestimmt. Dabei ist die Reaktionszeit variabel und ist abhängig von den Reaktionsbedingungen und dem gewünschten Grad der Aktivierung. Nach der Aktivierung wird der Katalysator zur Entfernung des Aluminats mit kalter Lauge, danach mit Wasser gewaschen und anschließend unter Wasser gelagert.
   Andere Zusammensetzungen können im Herstellungsschritt a) durch die entsprechende Wahl der Metallmengen analog produziert werden.

Bevorzugt wird der Katalysator in der beschriebenen Reihenfolge hergestellt. Die Aktivierung des Katalysators kann aber auch vor der Herstellung der Hohlkugeln erfolgen.

Es ist möglich, das erfindungsgemäße Verfahren in einer Stufe oder in mindestens zwei oder mehreren Stufen durchzuführen.

Wird das Verfahren in einer Stufe durchgeführt, wird Isophoronnitril direkt in Anwesenheit von Ammoniak, Wasserstoff, eines Katalysator und gegebenenfalls weiteren Zusätzen und in An- oder Abwesenheit von organischen Lösungsmitteln aminierend hydriert.

Der Begriff "in mindestens zwei oder in mehreren Stufen" bedeutet, dass zunächst in einem separaten Reaktor oder Reaktorabschnitt Isophoronnitril ganz oder teilweise in Isophoronnitrilimin umgewandelt wird, und dieses Isophoronnitrilimin als Reinsubstanz oder im Gemisch mit anderen Komponenten, wie zum Beispiel nicht umgesetztes Isophoronnitril, unter Anwesenheit von mindestens Ammoniak und Wasserstoff und eines Katalysators aminierend hydriert wird.

Eine bevorzugte Ausführungsform des erfindungsgemäßen Verfahrens zur Herstellung von IPDA ist ein zweistufiger Prozess: In der ersten Stufe wird zumindest ein Teil des eingesetzten IPN bei An- oder Abwesenheit eines Iminierungskatalysators und/oder von Lösungsmitteln durch Reaktion mit Ammoniak in Isophoronnitrilimin umgewandelt. Der Umsatz von IPN zu IPNI sollte nach der Iminierung größer 80 %, bevorzugt größer 90 %, besonders bevorzugt größer 95 % betragen. In der zweiten Stufe wird das Reaktionsprodukt der ersten Stufe, so wie es anfällt oder nach einer Weiterbehandlung und/oder Zugabe weiteren Ammoniaks, unter Anwesenheit von mindestens Ammoniak und Wasserstoff und in An- oder Abwesenheit eines organischen Lösungsmittel bei einer Temperatur von 20 bis 150 °C, bevorzugt 40 bis 130 °C, und einem Druck von 0,3 bis 50 MPa, bevorzugt 5 bis 30 MPa, an Hydrierkatalysatoren aminierend hydriert.

In einer weiteren bevorzugten Ausführungsform erfolgt die Umsetzung von IPN zu IPDA in drei voneinander getrennten Reaktionsräumen. In dem ersten Reaktionsraum erfolgt die Umsetzung von IPN zu Isophoronnitrilimin mit überschüssigem Ammoniak an Iminierungskatalysatoren bei Temperaturen zwischen 20 und 150 °C und Drücken zwischen 5 und 30 MPa. Im zweiten Reaktionsraum werden die entstandenen Reaktionsprodukte mit Wasserstoff in Gegenwart von überschüssigem Ammoniak an Hydrierkatalysatoren bei Temperaturen zwischen 20 und 130 °C und Drücken von 5 bis 30 MPa hydriert. Im dritten Reaktionsraum werden die entstanden Reaktionsprodukte an den erfindungsgemäß einzusetzenden Katalysatoren bei Temperaturen zwischen 100 und 160 °C und Drücken von 5 bis 30 MPa hydriert.

Um die Gleichgewichtseinstellung der Iminierungsreaktion zu beschleunigen ist es zweckmäßig, einen Iminierungskatalysator zu verwenden. Hierzu können die nach dem Stand der Technik bekannten Iminierungskatalysatoren verwendet werden. Geeignete Katalysatoren sind beispielsweise anorganische oder organische Ionenaustauscher (siehe EP 042 119), geträgerte Heteropolysäuren (siehe DE 44 26 472), acide Metalloxide, insbesondere Aluminiumoxid und Titandioxid (siehe EP 449 089) Sulfonsäuregruppen enthaltende Organopolysiloxane (DE 196 27 265.3) und saure Zeolithe sowie Aktivkohle (EP 061 137). Bei Verwendung eines Iminierungskatalysators kann die Reaktionstemperatur zwischen 10 und 150 °C, vorzugsweise zwischen 30 und 130 °C und ganz besonders bevorzugt zwischen 40 und 100 °C liegen. Der Druck liegt zwischen dem Eigendruck der Mischung und 50 MPa. Bevorzugt wird die Iminierungsreaktion bei dem Druck durchgeführt, bei dem auch die anschließende reduktive Aminierung durchgeführt wird.

Obwohl die Iminierung von Isophoronnitril mit flüssigem Ammoniak bevorzugt ohne Zugabe weiterer Lösungsmittel durchgeführt wird, kann auch in Anwesenheit zusätzlicher Lösungsmittel gearbeitet werden. Geeignet sind einwertige Alkohole mit 1 bis 4 C-Atomen, insbesondere Methanol sowie Ether, besonders THF, MTBE und Dioxan.

In der Iminierungsstufe werden pro Mol eingesetztem IPN zwischen 1 und 500 Mol, bevorzugt 5 und 200 Mol, besonders bevorzugt zwischen 5 und 100 Mol Ammoniak eingesetzt. Typische Katalysatorbelastungen liegen im Bereich von 0,01 bis 10 kg IPN je kg Katalysator und Stunde, bevorzugt 0,5 bis 10 und besonders bevorzugt 0,5 bis 5 kg IPN je kg Katalysator und Stunde.

Bei der Iminierung in Gegenwart eines Iminierungskatalysators kann der Katalysator in Form eines Suspensionskatalysators oder Festbettkatalysators vorliegen. Vorteilhaft ist die Verwendung von Festbettkatalysatoren. In einer besonders bevorzugten Ausführungsform werden IPN und Ammoniak kontinuierlich von unten nach oben durch ein mit Iminierungskatalysator gefülltes Reaktionsrohr geleitet.

Die Hydrierung erfolgt in Festbettreaktoren. Geeignete Reaktortypen sind z. B. Schachtöfen, Hordenreaktoren oder Rohrbündelreaktoren.

Die Hydrierung erfolgt üblicherweise bei Temperaturen zwischen 20 und 150 °C, bevorzugt 40 und 130 °C, und Drücken von 0,3 bis 50 MPa, bevorzugt 5 bis 30 MPa. Es ist auch möglich, die Hydrierung in Gegenwart der bereits bei der Iminierungsstufe genannten Lösungsmittel durchzuführen. Der wesentliche Vorteil bei Verwendung eines Lösungsmittels liegt darin, dass die Hydrierung bei niedrigeren Drücken zwischen 0,3 und 10 MPa durchgeführt werden kann.

Der für die Hydrierung erforderliche Wasserstoff kann dem Reaktor entweder im Überschuss, beispielsweise mit bis zu 10000 Moläquivalenten, zugeführt werden, oder nur in einer solchen Menge, dass der durch Reaktion verbrauchte Wasserstoff sowie der Teil des Wasserstoffs, der eingelöst im Produktstrom den Reaktor verlässt, nachgeführt wird. Bei kontinuierlicher Fahrweise kann der Wasserstoff im Gleich- oder Gegenstrom zugeführt werden.

In einer bevorzugten Ausführungsform erfolgt die Hydrierung in flüssigem Ammoniak als Lösungsmittel. Pro Mol IPN werden zwischen 1 und 500 Mol, bevorzugt 5 und 200 Mol, besonders bevorzugt zwischen 5 und 100 Mol Ammoniak verwendet. Zweckmäßigerweise verwendet man mindestens die Ammoniakmenge, die bei der vorgelagerten Iminierung eingestellt wurde. Der Ammoniakanteil kann aber auch vor der Hydrierung durch Zugabe von zusätzlichem Ammoniak auf den gewünschten Wert erhöht werden.

Das erforderliche Volumen der einzusetzenden Hydrierkatalysatoren richtet sich nach dem vom Betriebsdruck, der Temperatur, der Konzentration und der Katalysatoraktivität abhängigen LHSV-Wert, (liquid hourly space velocity) der eingehalten werden muss, um eine möglichst vollständige Hydrierung des eingesetzten IPN zu gewährleisten. Üblicherweise liegt der LHSV-Wert bei der Verwendung des bevorzugt einzusetzenden Gemisches aus IPN, Ammoniak und Wasserstoff zwischen 0,5 und 5 Liter IPN/Ammoniak-Mischung pro Liter Katalysator und Stunde, bevorzugt zwischen 1 und 4 I_{Lsg} I_{Kat}⁻¹ h⁻¹.

Es ist bevorzugt, dass die einzusetzenden Hydrierkatalysatoren vor ihrem Einsatz in der Hydrierung zunächst mit Ammoniak konditioniert werden. Dazu werden die Katalysatoren mit Ammoniak oder mit Mischungen aus Ammoniak und einem oder mehrerer Lösungsmittel in Kontakt gebracht. Bevorzugt erfolgt die Konditionierung nach Einbau der Katalysatoren im Hydrierreaktor, sie kann aber auch vor Einbau der Katalysatoren erfolgen. Zur Konditionierung werden zwischen 0,2 und 3, bevorzugt 0,5 und 2 m³ Ammoniak pro m³ Katalysator und Stunde eingesetzt. Üblicherweise wird bei Temperaturen zwischen 20 und 150 °C, bevorzugt 40 bis 130 °C, gearbeitet. Besonders bevorzugt wird eine Temperaturrampe durchfahren, bei der der Katalysator beginnend bei mäßig erhöhter Temperatur, bevorzugt zwischen 20 und 50 °C, langsam bis auf die später für die Hydrierung gewünschte Reaktionstemperatur, bevorzugt 20 bis 150 °C, aufgeheizt wird. Die Konditionierung wird bevorzugt in Gegenwart von Wasserstoff durchgeführt, wobei der Partialdruck des verwendeten Wasserstoffs im Reaktor den Bereich von 0,1 bis 50 MPa, bevorzugt 5 bis 40 MPa, besonders bevorzugt 10 bis 30 MPa umfasst. Die Zeitspanne der Konditionierung ist abhängig von der verwendeten Ammoniakmenge und liegt bevorzugt zwischen 1 und 48 h, besonders bevorzugt zwischen 12 und 24 h.

In dem bevorzugten zweistufigen Prozess wird das Isophoronnitrilimin enthaltende Gemisch in Gegenwart des Hydrierkatalysators in der zweiten Stufe hydriert. Das der Hydrierstufe zugeführte Gemisch kann unmittelbar jenes sein, welches bei der Iminierung von IPN mit Ammoniak in der ersten Stufe anfällt, oder wie es nach Zugabe oder Entfernung von Komponenten, wie z. B. Ammoniak, organischen Lösungsmitteln, Basen, Cyanidsalze, Blausäure und/oder Wasser, erhalten wird. Bevorzugt wird die Hydrierung kontinuierlich in Festbettreaktoren durchgeführt, die in Riesel- oder Sumpffahrweise betrieben werden können. Geeignete Reaktortypen sind z.B. Schachtöfen, Hordenreaktoren oder Rohrbündelreaktoren. Es ist auch möglich, für die Hydrierung mehrere Festbettreaktoren hintereinander zu schalten, wobei jeder der Reaktoren wahlweise in Rieselbett- und Sumpffahrweise betrieben wird.

Außer den zuvor genannten Bestandteilen des der Iminierungsstufe zuzuführenden Gemisches kann dieses zusätzlich höher oder niedriger als IPDA siedende Fraktionen aus der destillativen Aufarbeitung des aus dem Rieselbettreaktor abgezogenen Reaktionsgemisches enthalten. Derartige Fraktionen können außer Resten an IPDA auch solche Nebenprodukte enthalten, aus denen sich unter Reaktionsbedingungen erneut IPDA bildet. Besonders vorteilhaft ist es, die oberhalb IPDA siedende Fraktion, welche außer Resten an IPDA 2-Aza-4,6,6-trimethylbicyclo[3.2.1]-octan als Hauptprodukt enthält, zurückzuführen. Ebenfalls besonders vorteilhaft ist es, nicht vollständig umgesetztes IPN, insbesondere Isophoronaminonitril enthaltende Fraktionen, zurückzuführen. Die Rückführungen können auch, falls dieses gewünscht ist, unmittelbar dem der Hydrierstufe zuzuführenden Reaktionsgemisch zugesetzt werden.

Bei der Hydrierung von IPN bzw. Isophoronnitrilimin können zwei unterschiedliche Stereoisomere gebildet werden. Durch die Wahl eines Temperaturprofils im Hydrierschritt lässt sich das Isomerenverhältnis beeinflussen. Es ist z. B. möglich, ein IPN oder Isophoronnitrilimin enthaltendes Gemisch zunächst bei einer Temperatur zwischen 20 und 90 °C teilweise zu hydrieren, und anschließend die Reaktion in einem zweiten Schritt in einem Temperaturbereich zwischen 90 und 150 °C zu vervollständigen. Durch die Einhaltung relativ niedriger Reaktionstemperaturen im 1. Schritt kann die Selektivität zu Gunsten des cis-Isomeren verschoben werden. Die Einhaltung relativ niedriger Reaktionstemperaturen zu Beginn der Reaktion hat zudem den Vorteil, dass das thermisch labile Isophoronnitrilimin besonders schonend hydriert wird und dadurch Nebenreaktionen unterbunden werden. Das intermediär gebildete Isophoronaminonitril ist thermisch deutlich stabiler und kann daher bei höheren Temperaturen hydriert werden, ohne dass weitere Nebenreaktionen befürchtet werden müssen. Zu den unerwünschten Nebenreaktionen zählt auch die Abspaltung von HCN. Nach dem erfindungsgemäßen Verfahren wirkt sich eine gewisse Cyanidionenkonzentration positiv auf die Selektivität der Hydrierstufe aus. Dieser Effekt tritt verstärkt dann zu Tage, wenn die Cyanidionen schon von Anfang an in der Hydrierstufe vorliegen und nicht erst während der Hydrierung entstehen. Daher ist eine Abspaltung von HCN während der Hydrierstufe zu verhindern.

Die Realisierung des gewünschten Temperaturprofils kann beispielsweise durch die Hintereinanderschaltung von zwei oder mehr getrennt voneinander beheizbaren Reaktoren erfolgen. Es ist aber auch möglich, ein ansteigendes Temperaturprofil in nur einem Hydrierreaktor zu realisieren. Besonders bevorzugt erfolgt die Durchführung der Hydrierreaktion in einem adiabatisch betriebenen Rieselbettreaktor, bei dem das Reaktionsgemisch dem Reaktor bei Temperaturen zwischen 20 und 90 °C zugeführt wird, und aufgrund der auftretenden und durch das Reaktionsgemisch aufgenommenen Reaktionswärme zwischen 90 und 150 °C wieder verlässt.

Das die Hydrierung verlassende Reaktionsgemisch wird mit den üblichen Methoden weiter aufgereinigt, um ein IPDA mit der gewünschten Qualität zu erhalten. Dabei können alle gängigen Trennmethoden wie z. B. Destillation, Entspannungsverdampfung, Kristallisation, Extraktion, Sorption, Permeation, Phasentrennung oder Kombinationen aus den genannten zum Einsatz kommen. Die Aufreinigung kann kontinuierlich, absatzweise, ein- oder mehrstufig, im Vakuum oder unter Druck durchgeführt werden. Mögliche Komponenten, die beispielsweise bei der weiteren Aufreinigung abgetrennt werden, sind Wasserstoff, Ammoniak, Wasser sowie die bei der Herstellung von IPDA aus IPN anfallenden Nebenprodukte wie z. B. hydrierte HCN-Eliminierungsprodukte oder Verunreinigungen des IPN, methylierte Nebenprodukte und/oder unvollständig hydrierte Zwischenprodukte.

Bevorzugt wird die Aufreinigung durch Destillation unter Druck und/oder im Vakuum in mehreren Schritten erreicht. Hierfür lassen sich beliebige Destillationskolonnen mit und ohne Einbauten wie z. B. Dephlegmatoren, Trennwänden, ungeordnete Einbauten oder Schüttungen, geordnete Einbauten oder Packungen, Böden mit oder ohne Zwangsführung verwenden.

In einem ersten Schritt werden insbesondere Wasserstoff, Inertgase, Ammoniak, leicht siedende Verunreinigungen und ggf. auch Wasser in einer oder mehreren Destillationskolonnen ganz oder teilweise abgetrennt. Dabei erfolgt die Abtrennung bevorzugt bei einem Druck, der kleiner ist als im Reaktionsschritt. Erfolgt die Abtrennung in mehreren Destillationsschritten, so ist es vorteilhaft, das der Druck stufenweise abgesenkt wird. Ganz besonders bevorzugt erfolgt die Abtrennung oberhalb 1 bar und mit Sumpftemperaturen von 0 bis 200 °C. Der Einsatz eines Stripgases zur Entfernung von leicht siedenden Verunreinigungen kann vorteilhaft sein. Insbesondere Ammoniak und Wasserstoff und Anteile der leicht siedenden Verunreinigungen können ganz oder zum Teil in den Prozess (Reaktion) zurückgeführt werden. Die leicht siedenden Verunreinigungen und gegebenenfalls Anteile von Wasserstoff und Ammoniak werden der thermischen Verwertung zugeführt.

In einem zweiten Schritt werden weitere leicht siedende Verunreinigungen, Wasser und schwer siedende Verunreinigungen ganz oder teilweise abgetrennt. Dies kann in einer oder mehreren Destillationskolonnen erfolgen. Dabei kann Wasser zusammen mit organischen, leicht siedenden Verunreinigungen und gegebenenfalls Anteilen an IPDA über Kopf der Kolonne abdestilliert werden und nach Kondensation in eine wässrige und eine organische Phase getrennt werden. Hierbei kann die organische Phase teilweise als Rückfluss in die Kolonne zurückgeführt werden. Wird der zweite Schritt der Destillation in einer einzigen Kolonne durchgeführt (z. B. einer Trennwandkolonne), so wird das IPDA mit der gewünschten Reinheit über einen Seitenstrom entnommen, während die schwer siedenden Verunreinigungen im Sumpf der Kolonne anfallen. Wird die Trennung aber zwei- oder mehrstufig durchgeführt, so wird das IPDA am Kopf einer Kolonne erhalten. Die Abtrennung der leicht und schwer siedenden Verunreinigungen und von Wasser erfolgt bevorzugt im Vakuum zwischen 100 Pa und 0,0999 MPa und Sumpftemperaturen von 50 bis 300 °C. Sämtliche Nebenkomponenten können der thermischen Verwertung zugeführt werden.

### Beispiele

### Beispiel A: Herstellung des Katalysators:

### Herstellung der Legierung

Die Herstellung der Legierung erfolgt in einem Induktionsofen. Es werden dabei die Metalle in den entsprechenden Mengen bei 1500°C geschmolzen. Die fertige Schmelze wird für die Weiterverarbeitung zu Barren gegossen.

### Herstellung der Pulver

Die Legierungsbarren werden über einen Backenbrecher vorzerkleinert und über eine Kugelmühle weiter gemahlen. Durch einen Siebschritt wird die gewünschte Größenverteilung der Granulate durch die Wahl der entsprechenden Siebe erhalten.

### Beispiel 1: Herstellung der Hohlkugeln

Durch Suspendieren von 3179 g CoAlCrNi Legierung (Korngrößenverteilung < 200 µm) in 2861 g einer wässerigen Lösung mit einem Gehalt an ca. 2 Gew.-% Polyvinylalkolhol wurde eine Beschichtungslösung hergestellt. Diese Suspension wurde sodann auf 1.500 ml Polystyrolkugeln mit einem Durchmesser um ca. 1,8 mm aufgesprüht, während diese in einem nach oben gerichteten Luftstrom suspendiert waren.

1,5 Liter dieser Kugeln wurden mit einer Legierungslösung bestehend aus 3169 g CoAlCrNi Legierung suspendiert in 2852 g einer wässerigen Lösung mit einem Gehalt an ca. 2 Gew.-% Polyvinylalkolhol weiter beschichtet.

Nach dem Beschichten der Polystyrolkugeln mit den zuvor erwähnten Lösungen wurden die Kugeln bis 500 °C erwärmt, um das Polystyrol herauszubrennen. Die CoAlCrNi Hohlkugeln wurden sodann bis 900°C erwärmt.

Die Hohlkugeln wurden nach Abkühlen in einer 20 Gew.-%igen Natronlauge 70 Minuten bei 90°C aktiviert. Die erhaltenen aktivierten Hohlkugeln hatten einen Durchmesser von 2,5 bis 5,5 mm und eine Manteldicke von 600 bis 1000 µm.

Der eingesetzte Katalysator weist nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:
- Cobalt:: 55,6 Gew.-%
- Aluminium:: 20,9 Gew.-%
- Chrom:: 1,1 Gew.-%
- Nickel:: 1,7 Gew.-%
- Sauerstoff: 20,8 Gew.-%

### Beispiel 2

Die Katalysatoren werden bei der Herstellung von 3-Aminomethyl-3,5,5-Trimethylcyclohexylamin (Isophorondiamin, IPDA) aus 3-Cyano-3,5,5-Trimethylcyclohexanon (Isophoronnitril, IPN) in einem zweistufigen Verfahren auf ihr katalytische Wirksamkeit getestet.

In der ersten Stufe wird dabei Isophoronnitril in Anwesenheit eines Iminierungskatalysators mit Ammoniak zumindest teilweise zu 3-Cyano-3,5,5-Trimethylcyclohexanimin überführt und in der zweiten Stufe an einem Hydrierungskatalysator bei einer Temperatur von 60 bis 100°C und einem Druck von 250 bar mit Wasserstoff unter Anwesenheit von Ammoniak aminierend hydriert. Jede Stufe der Herstellung wird in einem separaten Reaktor durchgeführt. Beide Reaktoren sind jedoch hintereinander geschaltet und werden einzeln temperiert.

Der Hydrierreaktor wird mit 62 ml des zu prüfenden Katalysators befüllt. Die Einsatzlösung aus IPN (14 Gew.-%) und Ammoniak (86 Gew.-%) wird mit einem Massenstrom von 165 ml/h von oben nach unten durch das Reaktionsrohr gepumpt. Der Wasserstoff wird separat mit einem Volumenstrom von 40 l/h ebenfalls von oben zugegeben. Die Produktflüssigkeit wird unter dem Reaktor in einem Abscheidegefäß aufgefangen. Die gesammelte Produktmischung wird auf IPDA und entsprechende Nebenkomponenten gaschromatographisch untersucht. Die Ergebnisse sind in Tabelle 1 aufgeführt.

**Tabelle 1**

| Temperatur | Ausbeute IPDA/GC-% | Umsatz / % |
|---|---|---|
| 100 °C | 97,6 | 99,9 |
| 80 °C | 96,0 | 98,1 |
| 60 °C | 71,9 | 75,5 |

### Langzeitstabilität

In der ersten Stufe wird Isophoronnitril in Anwesenheit eines Iminierungskatalysators mit Ammoniak zumindest teilweise zu 3-Cyano-3,5,5-Trimethylcyclohexanimin überführt und in der zweiten Stufe an einem Hydrierungskatalysator bei einer Temperatur von 100°C und einem Druck von 250 bar mit Wasserstoff unter Anwesenheit von Ammoniak aminierend hydriert. Jede Stufe der Herstellung wird in einem separaten Reaktor durchgeführt. Beide Reaktoren sind jedoch hintereinander geschaltet und werden einzeln temperiert.

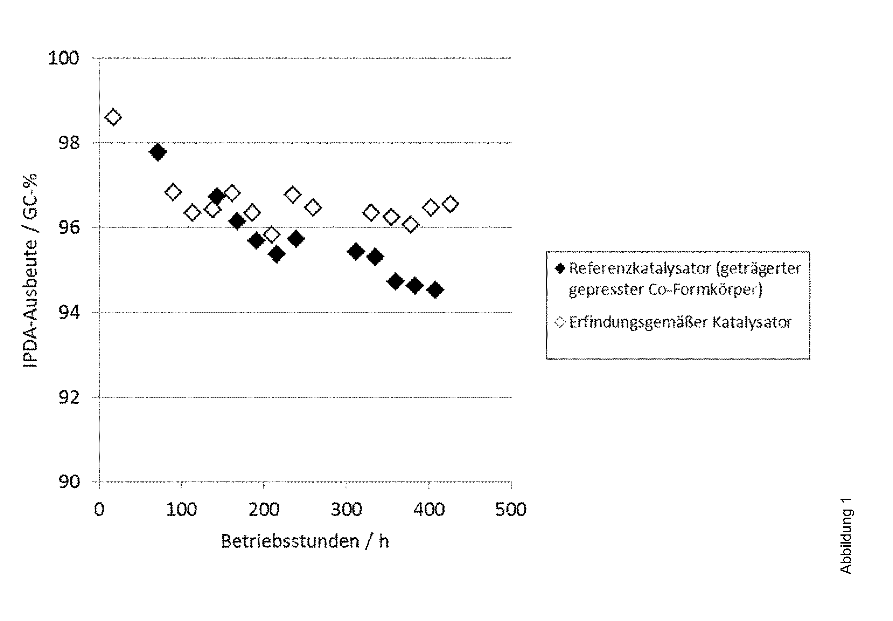

Für die Überprüfung der Langzeitstabilität wird der Hydrierreaktor mit 6 l des zu prüfenden Katalysator befüllt. Die Einsatzlösung aus IPN (24 Gew.-%) und Ammoniak (76 Gew.-%) wird mit einem Volumenstrom von 10 l/h von oben nach unten durch das Reaktionsrohr gepumpt. Zusätzlich wird Wasserstoff ebenfalls von oben zugegeben. Die Produktflüssigkeit wird unter dem Reaktor in einem Abscheidegefäß aufgefangen. Die gesammelte Produktmischung wird auf IPDA und entsprechende Nebenkomponenten gaschromatographisch untersucht. Die Ergebnisse sind in Abbildung 1 aufgeführt.

Erfindungsgemäßer Katalysator: Co-Hohlkugeln der Zusammensetzung wie oben in Beispiel 1 beschrieben.

Referenzkatalysator: Geträgerter gepresster Co-Katalysator-Formkörper.

## Patentansprüche

1. Verfahren zur Herstellung von Isophorondiamin, **dadurch gekennzeichnet, dass**
A) Isophoronnitril direkt in einer Stufe in Anwesenheit von Ammoniak, Wasserstoff, eines Hydrierkatalysators und gegebenenfalls weiteren Zusätzen und in An- oder Abwesenheit von organischen Lösungsmitteln aminierend zu Isophorondiamin hydriert wird;
oder
B) Isophoronnitril in mindestens zwei oder mehreren Stufen zu Isophorondiamin umgesetzt wird, wobei zunächst Isophoronnitril ganz oder teilweise in Isophoronnitrilimin umgewandelt wird, und dieses Isophoronnitrilimin als Reinsubstanz, oder im Gemisch mit anderen Komponenten und/oder Isophoronnitril, unter Anwesenheit von mindestens Ammoniak, Wasserstoff und eines Katalysators aminierend zu Isophorondiamin hydriert wird;
wobei der Katalysator die folgenden Eigenschaften aufweist:
I. Der Katalysator weist nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:
Cobalt: 55 bis 95 Gew.-%
Aluminium: 5 bis 45 Gew.-%
Chrom: 0 bis 3 Gew.-%
Nickel: 0 bis 7 Gew.-%
und
II. Der Katalysator liegt in Form von Hohlkugeln vor mit Durchmessern von 1 bis 8 mm.

2. Verfahren zur Herstellung von Isophorondiamin nach Anspruch 1, **dadurch gekennzeichnet, dass**
I. Der Katalysator nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle, aufweist:
Cobalt: 55 bis 90 Gew.-%
Aluminium: 5 bis 44,5 Gew.-%
Chrom: 0,5 bis 5 Gew.-%

3. Verfahren zur Herstellung von Isophorondiamin nach Anspruch 1, **dadurch gekennzeichnet, dass**
I. Der Katalysator nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle, aufweist:
Cobalt: 55 bis 88 Gew.-%
Aluminium: 5 bis 44,5 Gew.-%
Nickel: 0,5 bis 7 Gew.-%

4. Verfahren zur Herstellung von Isophorondiamin nach Anspruch 1, **dadurch gekennzeichnet, dass**
I. Der Katalysator nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle, aufweist:
Cobalt: 55 bis 85 Gew.-%
Aluminium: 5 bis 43,5 Gew.-%
Chrom: 0,5 bis 3 Gew.-%
Nickel: 1 bis 7 Gew.-%

5. Verfahren zur Herstellung von Isophorondiamin nach Anspruch 1, **dadurch gekennzeichnet, dass**
I. Der Katalysator nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle, aufweist:
Cobalt: 57 bis 84 Gew.-%
Aluminium: 10 bis 40 Gew.-%
Chrom: 1 bis 2 Gew.-%
Nickel: 2 bis 4 Gew.-%

6. Verfahren zur Herstellung von Isophorondiamin nach mindestens einem der vorherigen Ansprüche 1-5, **dadurch gekennzeichnet, dass**
der Durchmesser des Katalysators, also der Hohlkugeln, von 2,5 bis 5,5 Millimeter (mm) variiert,
und/oder
der Durchmesser des Katalysators, also der Hohlkugeln, von 3 bis 8 Millimeter (mm) variiert, und/oder
die Durchmesser des Katalysators, also der Hohlkugeln, von 1 bis 3 Millimeter (mm) variiert.

7. Verfahren zur Herstellung von Isophorondiamin nach Anspruch 1, **dadurch gekennzeichnet, dass** der Katalysator nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle, aufweist:
Cobalt: 57 bis 84 Gew.-%
Aluminium: 10 bis 40 Gew.-%
Chrom: 1 bis 2 Gew.-%
Nickel: 2 bis 4 Gew.-%
und mit
Durchmessern des Hohlkugel Katalysators, also der Hohlkugeln mit einer statistischen Verteilung zwischen 2,5 bis 5,5 Millimeter (mm),
und/oder
Durchmessern des Hohlkugel Katalysators, also der Hohlkugeln mit einer statistischen Verteilung zwischen 3,5 bis 6,5 Millimeter (mm),
und/oder
Durchmessern des Hohlkugel Katalysators, also der Hohlkugeln mit einer statistischen Verteilung zwischen 1 bis 3 Millimeter (mm),
und/oder
Durchmessern des Hohlkugel Katalysators, also der Hohlkugeln mit einer statistischen Verteilung zwischen 3 bis 8 Millimeter (mm),
wobei bis zu 10 Prozent der Partikel auch außerhalb des genannten Bereichs der genannten Untergrenze oder Obergrenze, aber auch jeweils bis zu 10 Prozent außerhalb des genannten Bereichs der genannten Untergrenze und Obergrenze, liegen können.

8. Verfahren zur Herstellung von Isophorondiamin nach mindestens einem der vorherigen Ansprüche, **dadurch gekennzeichnet, dass** die Katalysatoren zusätzlich Dotiermetalle enthalten, bevorzugt ausgewählt aus Mangan, Eisen, Vanadium, Tantal, Titan, Wolfram, Molybdän, Rhenium und/oder Metalle der Platingruppe.

9. Verfahren zur Herstellung von Isophorondiamin nach mindestens einem der vorherigen Ansprüche, **dadurch gekennzeichnet, dass** der Anteil an Dotiermetallen im Katalysator 0 bis 5 Gew.-% beträgt.

10. Verfahren zur Herstellung von Isophorondiamin nach mindestens einem der vorherigen Ansprüche, **dadurch gekennzeichnet, dass** der Anteil an Sauerstoff im Katalysator 0 bis 25 Gew.-% beträgt.

11. Verfahren zur Herstellung von Isophorondiamin nach mindestens einem der vorherigen Ansprüche, **dadurch gekennzeichnet, dass** der Katalysator aus einer oder mehreren Schichten aufgebaut ist und/oder die Schichten dabei sowohl aus dem gleichen und/oder unterschiedlichen Katalysatormaterialen, also Legierungspulvern, bestehen, und/oder, dass der Hohlkugelförmige Katalysator eine bimodale oder multimodale Verteilung in Bezug auf die Durchmesser der Hohlkugeln aufweist.

12. Verfahren zur Herstellung von Isophorondiamin nach mindestens einem der vorherigen Ansprüche, **dadurch gekennzeichnet, dass** in der ersten Stufe wird zumindest ein Teil des eingesetzten Isophoronnitrilimin bei An- oder Abwesenheit eines Iminierungskatalysators und/oder von Lösungsmitteln durch Reaktion mit Ammoniak in Isophoronnitrilimin umgewandelt, wobei der Umsatz von IPN zu IPNI nach der Iminierung größer 80 %, bevorzugt größer 90 %, besonders bevorzugt größer 95 % beträgt.

13. Verfahren zur Herstellung von Isophorondiamin nach mindestens einem der vorherigen Ansprüche, **dadurch gekennzeichnet, dass** in der zweiten Stufe das Reaktionsprodukt der ersten Stufe, so wie es anfällt oder nach einer Weiterbehandlung und/oder Zugabe weiteren Ammoniaks, unter Anwesenheit von mindestens Ammoniak und Wasserstoff und in An- oder Abwesenheit eines organischen Lösungsmittel bei einer Temperatur von 20 bis 150 °C, bevorzugt 40 bis 130 °C, und einem Druck von 0,3 bis 50 MPa, bevorzugt 5 bis 30 MPa, an Hydrierkatalysatoren aminierend hydriert wird.

14. Verfahren zur Herstellung von Isophorondiamin nach mindestens einem der vorherigen Ansprüche, **dadurch gekennzeichnet, dass** die Umsetzung von IPN zu IPDA in drei voneinander getrennten Reaktionsräumen erfolgt, wobei in dem ersten Reaktionsraum die Umsetzung von IPN zu Isophoronnitrilimin mit überschüssigem Ammoniak an Iminierungskatalysatoren bei Temperaturen zwischen 20 und 150 °C und Drücken zwischen 5 und 30 MPa erfolgt, im zweiten Reaktionsraum die entstandenen Reaktionsprodukte mit Wasserstoff in Gegenwart von überschüssigem Ammoniak an Hydrierkatalysatoren bei Temperaturen zwischen 20 und 130 °C und Drücken von 5 bis 30 MPa hydriert werden, und im dritten Reaktionsraum die entstanden Reaktionsprodukte an Katalysatoren bei Temperaturen zwischen 100 und 160 °C und Drücken von 5 bis 30 MPa hydriert werden.

15. Verfahren zur Herstellung von Isophorondiamin nach mindestens einem der vorherigen Ansprüche, **dadurch gekennzeichnet, dass** die Iminierungsreaktion in Gegenwart mindestens eines Iminierungskatalysators erfolgt.

16. Verfahren zur Herstellung von Isophorondiamin nach mindestens einem der vorherigen Ansprüche, **dadurch gekennzeichnet, dass** die Iminierung von Isophoronnitril mit flüssigem Ammoniak ohne Zugabe weiterer Lösungsmittel durchgeführt wird.

17. Verfahren zur Herstellung von Isophorondiamin nach mindestens einem der vorherigen Ansprüche, **dadurch gekennzeichnet, dass** in der Iminierungsstufe pro Mol eingesetztem IPN zwischen 1 und 500 Mol, bevorzugt 5 und 200 Mol, besonders bevorzugt zwischen 5 und 100 Mol Ammoniak eingesetzt werden.

18. Verfahren zur Herstellung von Isophorondiamin nach mindestens einem der vorherigen Ansprüche, **dadurch gekennzeichnet, dass**
die Iminierung in Gegenwart eines Suspensionskatalysators oder Festbettkatalysators, bevorzugt mindestens eines Festbettkatalysators, durchgeführt wird.

19. Verfahren zur Herstellung von Isophorondiamin nach mindestens einem der vorherigen Ansprüche, **dadurch gekennzeichnet, dass** bei der Iminierung IPN und Ammoniak kontinuierlich von unten nach oben durch ein mit Iminierungskatalysator gefülltes Reaktionsrohr geleitet werden.

20. Verfahren zur Herstellung von Isophorondiamin nach mindestens einem der vorherigen Ansprüche, **dadurch gekennzeichnet, dass** der für die Hydrierung erforderliche Wasserstoff dem Reaktor entweder im Überschuss, bevorzugt mit bis zu 10000 Moläquivalenten, zugeführt wird, oder in einer solchen Menge, dass der durch Reaktion verbrauchte Wasserstoff sowie der Teil des Wasserstoffs, der eingelöst im Produktstrom den Reaktor verlässt, nachgeführt wird.

21. Verfahren zur Herstellung von Isophorondiamin nach mindestens einem der vorherigen Ansprüche, **dadurch gekennzeichnet, dass** die Hydrierung in flüssigem Ammoniak als Lösungsmittel durchgeführt wird, wobei. pro Mol IPN werden zwischen 1 und 500 Mol, bevorzugt 5 und 200 Mol, besonders bevorzugt zwischen 5 und 100 Mol Ammoniak eingesetzt werden.

22. Verfahren zur Herstellung von Isophorondiamin nach mindestens einem der vorherigen Ansprüche, **dadurch gekennzeichnet, dass** die Hydrierkatalysatoren vor ihrem Einsatz in der Hydrierung zunächst mit Ammoniak konditioniert werden.

23. Verfahren zur Herstellung von Isophorondiamin nach mindestens einem der vorherigen Ansprüche, **dadurch gekennzeichnet, dass** Hydrierung kontinuierlich in Festbettreaktoren erfolgt.

24. Verfahren zur Herstellung von Isophorondiamin nach mindestens einem der vorherigen Ansprüche, **dadurch gekennzeichnet, dass** die Hydrierung kontinuierlich in Festbettreaktoren, die in Rieselbettfahrweise oder Sumpffahrweise betrieben werden, durchgeführt wird.

25. Verfahren zur Herstellung von Isophorondiamin nach mindestens einem der vorherigen Ansprüche, **dadurch gekennzeichnet, dass** das die Hydrierung verlassende Reaktionsgemisch einstufig oder mehrstufig aufgereinigt wird, und das Isophorondiamin erhalten wird.

26. Verfahren zur Herstellung von Isophorondiamin nach mindestens einem der vorherigen Ansprüche, **dadurch gekennzeichnet, dass** das die Hydrierung verlassende Reaktionsgemisch in zwei Schritten aufgereinigt wird, wobei in einem ersten Schritt insbesondere Wasserstoff, Inertgase, Ammoniak, leicht siedende Verunreinigungen und gegebenenfalls Wasser in einer oder mehreren Destillationskolonnen ganz oder teilweise abgetrennt wird und in einem zweiten Schritt weitere leicht siedende Verunreinigungen, Wasser und schwer siedende Verunreinigungen ganz oder teilweise in Destillationskolonnen abgetrennt werden, und das Isophorondiamin erhalten wird.

27. Katalysator zur Herstellung von Isophorondiamin,
wobei der Katalysator die folgenden Eigenschaften aufweist:
I. Der Katalysator weist nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:
Cobalt: 55 bis 95 Gew.-%
Aluminium: 5 bis 45 Gew.-%
Chrom: 0 bis 3 Gew.-%
Nickel: 0 bis 7 Gew.-%
und
II. Der Katalysator liegt in Form von Hohlkugeln vor mit Durchmessern von 1 bis 8 mm.

28. Verwendung eines Katalysators zur Herstellung von Isophorondiamin, **dadurch gekennzeichnet, dass**
A) Isophoronnitril direkt in einer Stufe in Anwesenheit von Ammoniak, Wasserstoff, eines Hydrierkatalysators und gegebenenfalls weiteren Zusätzen und in An- oder Abwesenheit von organischen Lösungsmitteln aminierend zu Isophorondiamin hydriert wird;
oder
B) Isophoronnitril in mindestens zwei oder mehreren Stufen zu Isophorondiamin umgesetzt wird, wobei zunächst Isophoronnitril ganz oder teilweise in Isophoronnitrilimin umgewandelt wird, und dieses Isophoronnitrilimin als Reinsubstanz, oder im Gemisch mit anderen Komponenten und/oder Isophoronnitril, unter Anwesenheit von mindestens Ammoniak, Wasserstoff und eines Katalysators aminierend zu Isophorondiamin hydriert wird;
wobei der Katalysator die folgenden Eigenschaften aufweist:
I. Der Katalysator weist nach der Aktivierung in seiner Gesamtheit die folgende Zusammensetzung auf in Gewichtsprozent (Gew.-%), wobei sich die Anteile zu 100 Gew.-% addieren, bezogen auf die enthaltenden Metalle:
Cobalt: 55 bis 95 Gew.-%
Aluminium: 5 bis 45 Gew.-%
Chrom: 0 bis 3 Gew.-%
Nickel: 0 bis 7 Gew.-%
und
II.
Der Katalysator liegt in Form von Hohlkugeln vor mit Durchmessern von 1 bis 8 mm.
